# EUROPEAN PATENT SPECIFICATION

(11) **EP 1 156 331 B1**
(45) Date of publication and mention of the grant of the patent: **17.06.2009**
(21) Application number: 01108773.1
(22) Date of filing: 06.04.2001
(51) Int. Cl.: G01N 33/569, G01N 33/554

(54) **Immunoassay for H. Pylori in fecal specimens using genus specific monoclonal antibody**
Immunoassay für H. pylori in Fäkalienproben mit Hilfe von gattungsspezifischen Antikörpern
Essais immunologique pour H. pylori en épreuves de fèces avec des anticorps genre-spécifiques

(30) Priority: 18.05.2000 US 574734
(43) Date of publication of application: 21.11.2001
(73) Proprietor: MERIDIAN BIOSCIENCE, INC., Cincinnati, OH 45244 (US)
(72) Inventor: Kozak, Kenneth James, Cincinnati, Ohio 45255 (US)
(74) Representative: Hoarton, Lloyd Douglas Charles

(56) References cited:
- EP-A- 0 806 667
- WO-A-99/61892
- DE-A- 19 847 628
- US-A- 5 932 430

## Description

### Background

This invention relates to a method for detecting *Helicobacter pylori* in fecal specimens.

*H. pylori* is a bacterium that is found in the upper gastrointestinal tract of humans which has been implicated in gastroduodenal diseases such as peptic ulcers, gastritis and other maladies. The bacterium was originally classified as a Campylobacter and then reclassified as a Helicobacter based on more detailed information regarding its ultrastructure and fatty acid composition.

A number of different techniques, both invasive and noninvasive, have been used to detect *H. pylori.* The invasive techniques involve gastric biopsies and cultures. The noninvasive techniques include a urea breath test, in which the patient is given C-13 or C-14 labeled urea with a beverage, and the detection of *H. pylori* antibody in sera using antigens in enzyme-linked immunosorbent assays (ELISA). Examples of the latter techniques are found in U.S. Patent 5,262,156 to Aleonohammad and European Patent Application 0 329 570 to Blaser.

Several major antigens have been identified and used in immunoassays in the detection of *H. pylori* antibodies. However, these assays have not exhibited the specificity and sensitivity that are desired in serodiagnosis. Newell, D.G., et al. Serodian. Immunother. Infec. Dis.,, 3:1-6 (1989). One problem with these immunoassays is cross-reactivity. Studies of the dominant antigens in *H. pylori,* in particular, the putative flagellar protein, which has a molecular weight of 60 Da, have shown that some of these antigen are not specific to *H. pylori* and also found in other bacteria such as *C. jeuni* and *C. coli.* A second problem that has been encountered in designing immunoassays for *H. pylori* is strain variation. Substantial differences in the antigens have been observed in different strains of *H. pylori.* These problems preclude designing an assay around the use of a single antigen. One approach that has been taken to improving the specificity and selectivity of antibody immunoassays for *H. pylori* has been to use a mixture of antigens from different *H. pylori* strains which mixture is enriched with certain antigen fragments. One ELISA which detects *H. pylori* antibodies in a blood sera is commercially available from Meridian Diagnostics. This assay uses a bacterial whole cell lysate as the antigen.

There are certain disadvantages to using an ELISA which employs antigens to detect the presence of *H. pylori* antibodies. In particular, the antibody titer in human sera remains high for a prolonged time (in some cases as much as six months) after the infection has been treated. Consequently, a positive test using this ELISA does not necessarily mean that the patient is currently infected and requires treatment for *H. pylori* infection. When confronted with a positive ELISA, treating physicians often order a gastric biopsy to confirm the presence of the bacteria before initiating antibiotic therapy. Therefore, the antigen-based ELISA does not eliminate the need for the invasive procedure. By contrast, if an immunoassay could be designed for detecting *H. pylori* antigen instead of the antibody, the need to obtain gastric biopsies to confirm infection could be reduced significantly because the antigen generally can not be detected in a patient within days of its treatment. Thus, there is a need for an ELISA which detects *H. pylori* antigen and, more particularly, there is a need for an ELISA for detecting *H. pylori* directly from fecal specimens. EP 0 806 667 and US 5 932 430 describe immunoassays for H. Pylori in faecal specimens using two polyclonal or monoclonal antibodies specific for H. pylori antigen.

While ELISA's for detecting microorganisms such as *C. difficile* and adenovirus in fecal specimens are known, in studies of patients with gastric biopsies which are positive for *H. pylori,* the bacteria ordinarily can not be cultured and isolated from the fecal specimens. This and the problems of cross reactivity and strain variation raised serious doubts that an ELISA could be designed that would be specific for *H. pylori* and sensitive enough to reliably detect *H. pylori* antigen directly from a fecal specimen.

### Summary of the Invention

The present invention provides a method for detecting *H. pylori* in fecal specimens which comprises:
(a) dispersing a fecal specimen suspected of carrying *H. pylori* in a sample diluent;
(b) contacting the fecal specimen in the diluent with a first antibody reactive with *H. pylori* antigen to form a complex of the antibody and the antigen;
(c) separating said specimen from said complex;
(d) exposing the complex to a second antibody reactive with said antigen, a portion of the antibody reacting with said complex, one of said first and second antibody being selected from the group consisting of polyclonal *H. pylori* antigen specific antibodies, a plurality of monoclonal *H. pylori* antigen specific antibodies and mixtures thereof and the other being a Helicobacter or Camplobacter genus specific monoclonal antibody, one of said first and second antibody being bound to a solid carrier and the other being labeled with a detection agent; and
(e) detecting the amount of the labeled antibody and in turn determining the presence of *H. pylori* antigen in said fecal specimen.

The immunoassay will typically be supplied in the form of a kit including a plate of antibody-coated wells, sample diluent, the labeled antibody, e.g., an enzyme-antibody conjugate, wash buffer and, in the case of an ELISA, a substrate solution. Alternatively, one or more of the following assays can be used to detect the presence of the *H. pylori* antigen: an enzyme-linked assay, a radioimmunoassay, a fluorescence immunoassay, a chemiluminescent assay, a lateral flow assay, an agglutination assay, a particulate-based assay, an immunoprecipitation assay and an immunoblotting assay.

### Detailed Description

The immunoassay of the present invention employs genus specific monoclonal antibody to Helicobacter or Camplobacter on one side of the assay and a *H. pylori* specific antibodies on the other side of the assay.

The genus specific monoclonal antibodies used herein can cross-react with different species and strains of Helicobacter or Camplobacter. Genus specific monoclonal antibody for Helicobacter or Camplobacter can be obtained commercially. Examples include the following monoclonal antibodies obtained from BIODESIGN International of Saco, Maine 04072 clone no P101 (IgG2a) (Cat. No. C45101M) clone no P102 (IgG2a) (Cat. No. C45102M)
clone no 7102 (IgG1) (Cat. No. C84102M)

Polyclonal *H. pylori* antigen specific antibodies can be obtained from the sera of a sensitized animal. Sensitization can be accomplished by injecting the antigen into an antibody producing species, typically a mammal and preferably a rabbit, goat or cow. Usually an initial injection is given followed by subsequent booster injections to maximize the response. Optimally, the injection regime is in multiple doses given to White New Zealand rabbits. The amount of antigen injected must be adequate to elicit a sufficient amount of antibody to be detectable. Antibody production is verified using a trial bleed and Indirect Fluorescent Assay. Two or more monoclonal *H. pylori* specific antibodies could be used as an alternative to using a polyclonal antibody. *H. pylori* specific monoclonal antibodies can be obtained.

*H. pylori* cells from ATCC strain 43504 have been found to be particularly useful in producing the polyclonal antibody. As previously mentioned, substantial strain variation has been observed in *H. pylori.* Differences in the organism have been observed in different geographic regions as well as dietary groups. However, antibodies obtained through sensitization using cells from strain 43504 have been found to be useful in detecting the organism across geographic regions and dietary groups. If necessary, for example, if it is found that the ELISA is not effective in detecting the organism in certain populations, cells from more than one strain of *H. pylori* could be used to produce the antibody.

Labeled antibodies are used to determine the presence of *H. pylori* antigen. The same labels used in known immunometric assays can be used to label the antibodies used in the present invention. Among these may be mentioned enzymes, such as alkaline phosphatase, horseradish peroxidase, etc., or fluorescent, luminescent or radioactive labels such as fluorescein, rhodamine, europium, luminol, acridium and radioactive isotopes I¹²⁵, etc., or colloidal particles such as gold and selenium, etc. More specifically, fluorogenic labels for detection by fluorimetry as described in U.S. Patent No. 3,940,475, enzymatic markers as described in U.S. Patent No. 3,654,090, and radioisotopes such as Iodine-125. One of the most common enzymatic markers is horseradish peroxidase (HRP) and alkaline phosphatase enzyme. Example 3 below illustrates labeling polyclonal antibodies with HRP.

The unlabeled antibody is used in the process of the present invention to extract the antigenic substance from the fecal specimen being tested. This antibody can be immobilized on any of the supports commonly used in immunometric assays. Among those that may be used are filter paper, plastic beads, polyethylene, polystyrene, polypropylene or other suitable test tube. The techniques for bonding antibodies to such materials are well known to those skilled in the art.

To prepare the fecal specimen for use in the assay, the specimen is dispersed in a protein-based sample diluent. The diluent being formulated and buffered to minimize cross-reactivity. As examples of sample diluents, mention can be made of fetal bovine serum, normal goat serum, guinea pig serum, horse serum, casein, albumin, gelatin, and bovine serum albumin (BSA). A dilution of one part fecal specimen and four parts diluent has been found to be useful. In addition to using the protein based additives, cross-reactivity can be reduced by the addition of detergents and increasing or decreasing pH or ionic strength of the diluent buffer. For example, many sample diluents contain Triton X-100 and/or Tween 20 at concentrations ranging between 0.05% and 2%. NaCl can be added in the ranges between 0-2.9% to alter the ionic strength of the buffer system. These changes lead to greater specificity by reducing the likelihood of weak or non-specific interactions from forming.

Cross-reactivity can also be addressed in the formulation of the *H. pylori* specific antibody solutions and the washes that are used in the assay. The *H. pylori* specific antibody can be provided in a buffered solution in conjunction with one of the protein sera mentioned previously. The washes used in the assay can be formulated and buffered by the addition of salts and surfactants to control cross-reactivity. A preferred wash for reducing cross-reactivity is a phosphate buffered saline solution.

The preparation of the antigen, production of the polyclonal antibodies and an ELISA are illustrated in more detail by the following non-limiting examples.

### Example 1

### Preparation of the Helicobacter pylori (H. pylori) antigen:

*H. pylori* (ATCC strain 43504) was streaked for isolation on Tryptic Soy Agar (TSA) supplemented with 5% defibrinated sheep blood. The plate was incubated at 37°C in a microaerophilic environment for 6-7 days. The resultant bacterial growth was evaluated by use of colony morphology, urease, catalase and oxidase reactions, and gram stain. Acceptable growth was subcultured to four TSA with sheep blood agar plates and grown at 37°C in a microaerophilic environment for 3-4 days.

Each plate was flooded with 5ml of 0.85% NaCl and the bacterial growth was harvested by a plate spreader. The bacteria were centrifuged at 10,000xg for 15 minutes at 2-8°C. Each pellet was resuspended in 3ml of 0.85% NaCl and combined to one centrifuge container. The bacterial suspension was centrifuged at 10,000xg for 15 minutes at 2-8°C. The pellet was resuspended and centrifuged as before. The final pellet was resuspended to 3% of the total original volume in 20mM phosphate buffer. The bacterial cells were transferred to an iced container and sonicated 5 times for 3 minutes at the maximum setting that does not cause foaming, resting for 30 seconds in between cycles. The sonicated bacterial cells were centrifuged at 57,000xg for 15 minutes at 2-8°C. The bacterial supernatant was collected and the pellet discarded.

### Example 2

### Production of Rabbit Polyclonal:

The bacterial supernatant obtained in Example 1 was diluted in equal parts with Freunds complete adjuvant (total immunogen is 1.0ml) to provide 1 x 10⁸ cells per ml. This solution was mixed thoroughly and 0.2-0.5ml of the solution was injected intramuscularly into the right hind leg and 0.1-0.25ml of the solution was injected subcutaneously into each of eight to ten sites on the back. Subsequent injections were one month apart using Freunds incomplete adjuvant and the injection sites were limited to subcutaneous back.

A trial bleed was taken after three months. The bleed was taken from the central ear vein one week after the third injection. This bleed was incubated overnight at 2-8°C. The next day the blood was centrifuged at 5,000xg for 15 minutes at room temperature. The supernatant was collected and the pellet discarded. The supernatant was tested by an Indirect Fluorescent Assay (IFA). The IFA was performed by placing 10ul of *H. pylori* suspension on glass slides and heat fixed. The slides were blocked with 3% bovine serum albumin (BSA) for 5 minutes then washed with a wash of phosphate buffered saline (PBS) and 0.5% Tween 20 (PBS/Tween wash). 50ul of the trial bleeds and normal rabbit serum, as a control, diluted 1:10 in phosphate buffered saline with sodium azide (PBSA) were added and incubated in a humid environment for 30 minutes. After washing with PBS/Tween wash, Goat anti-Rabbit conjugated to FITC (fluoroescein isothiocyanate) was diluted 1:10 in PBSA and 50ul was added to each well. The slides were incubated for 30 minutes in a dark humid environment. The slides were washed again. Fluorescence assay mounting media and a cover slip were added and viewed with a fluorescence microscope. Rabbits whose sera demonstrated a 4+ fluorescence intensity reading were then volume bled.

The volume bleed was obtained similarly to the trial bleed except 50ml was removed from each rabbit. The blood was incubated and centrifuged as the trial bleed was.

The total volume of sera was determined and an equal volume of phosphate buffer saline (PBS) was added. A 40% ammonium sulfate precipitation was performed to remove unnecessary protein and incubated at 2-8°C for 24 hours. The mixture was transferred to a centrifuge tube and centrifuged at 10,000xg for 30 minutes at room temperature. Resuspend the pellet in PBS to approximately one third of the original volume. The suspension was dialyzed against 200 times the total suspension volume of 0.0175M potassium phosphate, pH 6.5 at 2-8°C. After the dialysis, the suspension was centrifuged at 10,000xg for 20 minutes at room temperature. The supernatant was collected and the pellet was discarded.

A DEAE (diethylaminoethyl cellulose) column was equilibrated with 0.0175M potassium phosphate, pH 6.5 at room temperature. The supernatant was placed over the column and the effluent fractions collected. A protein concentration (OD₂₈₀) was determined and all fractions greater than 0.200 were pooled. The pooled antibody was tested in the ELISA.

### Example 3

### Horseradish peroxidase conjugation:

The conjugation used 10mg of DEAE purified rabbit anti-*H. pylori* antibody. The antibody was brought to a final volume of 2.5ml by concentration or by the addition of 10mM sodium bicarbonate pH 9.6. A PD-10 column (Pharmacia) was equilibrated with 10mM sodium bicarbonate pH 9.6. The antibody was added to the column and nine fractions of 1.0ml were taken. A protein concentration (OD₂₈₀E.O.=1.4) of each fraction was taken and those reading above 0.200 were pooled.

A separate PD-10 column was equilibrated with 1mM sodium acetate trihydrate pH 4.3. The minimum amount of Horseradish Peroxidase (HRP) used was 1.172mg HRP for each 1mg of antibody. 1-1/2 times the calculated minimum HRP was weighed out and added to 1.0ml of deionized water. A protein concentration (OD₄₀₃E.O.=2.275) was performed and HRP diluted to 10mg/ml with deionized water. 0.1M sodium m-periodate was added at a concentration of 0.2ml for every 4mg HRP. This reaction was allowed to proceed for 20 minutes at room temperature with gentle rocking. The reaction was stopped by the addition of 50ul of 2M ethylene glycol for every ml of HRP at 4mg. The HRP was eluted through the PD-10 with 1mM sodium acetate trihydrate pH 4.3.

The conjugation ratio was 1mg antibody to 1.172mg HRP. The antibody was adjusted with 10mM sodium bicarbonate pH 9.6 and the HRP with 1mM sodium acetate trihydrate pH 4.3. The two were combined in a dedicated flask and pH adjusted with 0.2M sodium bicarbonate, pH 9.6 to 9.6. Protected from light, the mixture was incubated for two hours on a rotator at 85-95rpm at room temperature. After two hours, 0.1ml of 4mg/ml of sodium borohydride was added for every 8mg of antibody. The new mixture was incubated at 4°C for two hours on a rotator. The conjugate was passed through a PD-10, equilibrated with PBS, and fractions containing the conjugate were collected. The fractions were pooled and concentrated to approximately 1.0ml. The concentrate was placed over a Sephracryl S-200 column equilibrated with PBS at a flow rate of 10ml/hr. 2.0ml fractions were collected and a protein concentration of both the antibody and the HRP were performed. The fractions with a simultaneous peak in both the OD₂₈₀ and OD₄₀₃ were pooled and concentrated to approximately 1.0mg/ml.

The invention also includes a so called "simultaneous" or "reverse" assay. A simultaneous assay involves a single incubation step as the antibody bound to the solid support and the labeled antibody are both added to the sample being tested at the same time. After the incubation is complete, the solid support is washed to remove the residual sample and uncomplexed labeled antibody. The presence of the labeled antibody associated with the solid support is then determined. A reverse assay involves the step wise addition first of a solution of labeled antibody to the fecal specimen followed by the addition of the unlabeled antibody bound to the support. After a second incubation, the support is washed in a conventional fashion to free it of the residual specimen and the unreacted labeled antibody.

So-called triple sandwich assays can also be used for detecting *H. pylori* in fecal specimens in accordance with the invention. Triple assays are known in the art and the basic methodology can be applied to the detection of *H*. *pylori* in fecal specimens. A triple assay is typically conducted by dispersing a fecal specimen suspected of carrying *H. pylori* in a sample diluent which minimizes cross-reactivity and adding the diluted sample to an immobilized genus specific monoclonal antibody. The sample is incubated to form the antibody-antigen complex. After washing excess specimen from the immobilized support, an *H. pylori* specific antibody known as a primary antibody and obtained from a species of an antibody producing animal is added to the antibody-antigen complex and incubated to form an antibody-antigen-antibody complex. After forming this complex and removing the unreacted antibody, the complex is reacted with an antibody known as a secondary antibody which is an antibody to the antibody producing species such as anti-(rabbit, cow or goat) immunoglobulin. The secondary antibody is labeled in a conventional manner, typically with an enzyme, and incubated with the antibody-antigen-antibody complex to form a triple antibody complex or sandwich. After removing the unreacted secondary antibody, the antigen is assayed in a conventional manner. In using an enzyme label, a substrate is added to the complex of the antigen and the three antibodies and the reaction of the substrate with the linked enzyme is monitored to determine the amount of the antigen present in the specimen. In the triple sandwich assay, as in the basic sandwich assay, the washes and the antibody solutions are formulated or buffered to control cross-reactivity as needed.

Having described the invention in detail and by reference to the preferred embodiments it will be apparent to those skilled in the art that modifications and variations are possible without departing from the scope of the invention as defined in the following appended claims.

## Claims

1. A process for the determination of *H. pylori* in a fecal specimen which comprises:
(a) dispersing a fecal specimen in a sample diluent;
(b) contacting the fecal specimen in the diluent with a first antibody to form a complex of the antibody and the antigen;
(c) separating said specimen and said complex;
(d) exposing the complex to a second antibody and a portion of the second antibody reacting with said complex, one of said first and second antibody being selected from the group consisting of polyclonal antibodies specific for *H. pylori* antigen, a plurality of monoclonal *H. pylori* antigen specific antibodies and mixtures thereof, and the other being a Helicobacter or Campylobacter genus specific monoclonal antibody, one of said first and second antibody being bound to a solid carrier and the other being labeled with a detection agent; and
(e) detecting the amount of the labeled antibody and in turn determining the presence of *H. pylori* antigen in said fecal specimen.

2. The process of claim 1 wherein the first antibody is bound to a solid carrier and the second antibody is labeled with a detection agent.

3. The process of claim 1 wherein the first antibody is labeled with a detection agent and the second is bound to a solid carrier.

4. The process of claim 1 wherein the sample diluent is a protein based diluent.

5. The process of claim 1 wherein said first antibody is said genus specific monoclonal antibody and said second antibody is Docket No. 041497-015 selected from the group consisting of polyclonal antibodies, a plurality of monoclonal antibodies and mixtures thereof.

6. The process of claim 4 wherein the sample diluent contains a protein selected from the group consisting of fetal bovine serum, normal goat serum, guinea pig serum, horse serum, casein, albumin, gelatin, and bovine serum albumin.

7. The process of claim 1 wherein after exposing the complex to the second antibody, the complex is washed with a buffer that reduces cross-reactivity or otherwise improves the specificity of the assay.

8. The process of claim 5 wherein the second antibody is a polyclonal antibody obtained by sensitizing an antibody-producing mammal with *H. pylori* cells.

9. The process of claim 3 wherein said detection agent is selected from the group consisting of alkaline phosphatase and beta galactosidase horseradish peroxidase.

10. The process of claim 7 wherein said wash is phosphate buffered saline.

11. A process for the determination of *H. pylori* in a fecal specimen which comprises:
(a) dispersing a fecal specimen in a diluent;
(b) contacting the fecal specimen in the diluent with a first antibody reactive with *H. pylori* antigen bound to a solid carrier and a second labeled antibody reactive with *H. pylori* to form a complex of the antibodies and the antigen, one of said first and second antibody being selected from the group consisting of polyclonal antibodies specific for H. pylori, a plurality of *H. pylori* antigen specific monoclonal antibodies and mixtures thereof and the other being a Helicobacter or Campylobacter genus specific monoclonal antibody;
(c) separating said specimen and said complex;
(d) detecting the labeled antibody and in turn determining the presence of *H. pylori* antigen in said fecal specimen.

12. A process for the determination of *H. pylori* in a fecal specimen which comprises:
(a) dispersing a fecal specimen in a sample diluent; (b) contacting the fecal specimen in the diluent with a Helicobacter or Campylobacter genus specific monoclonal antibody bound to a solid carrier to form a complex of the antibody and the antigen;
(c) separating said specimen and said complex;
(d) contacting the antibody-antigen complex formed in step (b) with a primary antibody specific for *H.* pylori antigen obtained from an antibody-producing species to produce an antibody-antigen-antibody complex;
(e) removing the primary antibody not present in the complex from step (c);
(f) contacting the antibody-antigen-antibody complex formed in step (e) with a secondary antibody, said secondary antibody being an antibody for the antibody-producing species, whereby said secondary antibody forms a complex with said antibody-antigen-antibody complex; and
(g) determining the presence of *H. pylori* antigen in said fecal specimen.

13. A kit for the determination of *H. pylori* in a fecal specimen including a plate of wells having bound thereto a genus specific monoclonal antibody for *H. pylori* antigen, a protein-based sample diluent and a plurality of labeled antibodies specific for *H. pylori* antigen.

## Patentansprüche

1. Verfahren für die Ermittlung von *H pylori* in einer Fäkalienprobe, welches umfässt:
(a) Dispergieren einer Fäkalienprobe in einem Probenverdünner;
(b) Berühren der Fäkalienprobe in dem Verdünner mit einem ersten Antikörper, um einen Komplex aus dem Antikörper und dem Antigen zu bilden;
(c) Trennen der besagten Probe und des besagten Komplexes;
(d) Aussetzen des Komplexes gegenüber einem zweiten Antikörper und einem Anteil des zweiten Antikörpers, der mit dem besagten Komplex reagiert, wobei der besagte erste oder zweite Antikörper aus der Gruppe ausgewählt ist, die aus für *H pylori*-Antigen spezifischen polyklonalen Antikörpern, einer Vielzahl monoklonaler *H pylori-*antigenspezifischer Antikörper und Mischungen davon besteht, und der andere ein Helicobacter- oder Campylobacter-gattungsspezifischer monoklonaler Antikörper ist, wobei der besagte erste oder zweite Antikörper an einen festen Trägerstoff gebunden und der andere mit einem Nachweismittel markiert ist; und
(e) Nachweisen der Menge des markierten Antikörpers und folglich Ermitteln des Vorhandenseins von *H pylori*-Antigen in der besagten Fäkalienprobe

2. Verfahren nach Anspruch 1, wobei der erste Antikörper an einen festen Trägerstoff gebunden und der zweite Antikörper mit einem Nachweismittel markiert ist

3. Verfahren nach Anspruch 1, wobei der erste Antikörper mit einem Nachweismittel markiert und der zweite an einen festen Trägerstoff gebunden ist.

4. . Verfahren nach Anspruch 1, wobei der Probenverdünner ein proteinbasierter Verdünner ist

5. Verfahren nach Anspruch 1, wobei der besagte erste Antikörper der besagte gattungsspezifische monoklonale Antikörper und der besagte zweite Antikörper aus der Gruppe ausgewählt ist, die aus polyklonalen Antikörpern, einer Vielzahl monoklonaler Antikörper und Mischungen davon besteht.

6. Verfahren nach Anspruch 4, wobei der Probenverdünner ein Protein enthält, das aus der Gruppe ausgewählt ist, welche aus fetalem bovinem Serum, normalem Ziegenserum, Meerschweinchenserum, Pferdeserum, Kasein, Albumin, Gelatine und bovinem Serumalbumin besteht

7. Verfahren nach Anspruch 1, wobei der Komplex nach dem Aussetzen des Komplexes gegenüber dem zweiten Antikörper mit einem Puffer gewaschen wird, welcher die Kreuzreaktivität verringert oder andernfalls die Spezifität des Assays verbessert

8. Verfahren nach Anspruch 5, wobei der zweite Antikörper ein polyklonaler Antikörper ist, der durch Sensibilisieren eines Antikörper produzierenden Säugetiers mit *H. pylori*-Zellen gewonnen wird

9. . Verfahren nach Anspruch 3, wobei das besagte Nachweismittel aus der Gruppe ausgewählt ist, welche aus alkalischer Phosphatase und Beta-Galaktosidase-Meerrettichperoxidase besteht

10. Verfahren nach Anspruch 7, wobei die besagte Waschflüssigkeit phosphatgepufferte Salzlösung ist.

11. Verfahren zur Ermittlung von *H. pylori* in einer Fäkalienprobe, welches umfässt:
(a) Dispergieren einer Fäkalienprobe in einem Verdünner;
(b) Berühren der Fäkalienprobe in dem Verdünner mit einem ersten Antikörper, der mit an einen festen Trägerstoff gebundenem *H pylori*-Antigen reagiert, und einem zweiten markierten Antikörper, der mit *H pylori* reagiert, um einen Komplex aus den Antikörpern und dem Antigen zu bilden, wobei der besagte erste oder zweite Antikörper aus der Gruppe ausgewählt ist, die aus für *H pylori* spezifischen polyklonalen Antikörpern, einer Vielzahl *H pylori*-antigenspezifischer monoklonaler Antikörper und Mischungen davon besteht, und der andere ein Helicobacter- oder Campylobacter-gattungsspezifischer monoklonaler Antikörper ist;
(c) Trennen der besagten Probe und des besagten Komplexes;
(d) Nachweisen des markierten Antikörpers und folglich Ermitteln des Vorhandenseins von *H pylori*-Antigen in der besagten Fäkalienprobe

12. Verfahren zur Ermittlung von *H pylori* in einer Fäkalienprobe, welches umfasst:
(a) Dispergieren einer Fäkalienprobe in einem Probenverdünner;
(b) Berühren der Fäkalienprobe in dem Verdünner mit einem Helicobacter- oder Campylobacter-gattungsspezifischen monoklonalen Antikörper, der an einen festen Trägerstoff gebunden ist, um einen Komplex aus dem Antikörper und dem Antigen zu bilden;
(c) Trennen der besagten Probe und des besagten Komplexes;
(d) Berühren des bei Schritt (b) gebildeten Antikörper-Antigen-Komplexes mit einem für *H pylori*-Antigen spezifischen primären Antikörper, der von einer Antikörper produzierenden Spezies gewonnen wird, um einen Antikörper-Antigen-Antikörper-Komplex herzustellen;
(e) Entfernen des primären Antikörpers, der in dem Komplex von Schritt (c) nicht vorhanden ist;
(f) Berühren des bei Schritt (e) gebildeten Antikörper-Antigen-Antikörper-Komplexes mit einem sekundären Antikörper, wobei der besagte sekundäre Antikörper ein Antikörper für die Antikörper produzierende Spezies ist, wobei der besagte sekundäre Antikörper einen Komplex mit dem besagten Antikörper-Antigen-Antikörper-Komplex bildet; und
(g) Ermitteln des Vorhandenseins von *H pylori*-Antigen in der besagten Fäkalienprobe

13. . Kit zur Ermittlung von *H pylori* in einer Fäkalienprobe, das eine Platte mit Vertiefungen enthält, an welche ein gattungsspezifischer monoklonaler Antikörper für *H pylori*-Antigen, ein proteinbasierter Probenverdünner und eine Vielzahl von für *H pylori-*Antigen spezifischen markierten Antikörpern gebunden sind.

## Revendications

1. Procédé d'analyse de présence de H. pylori dans un échantillon de selles, comprenant les étapes suivantes :
(a) dispersion d'un échantillon de selles dans un diluant d'échantillon ;
(b) mise de l'échantillon de selles dans le diluant au contact d'un premier anticorps afin de former un complexe de l'anticorps et de l'antigène ;
(c) séparation dudit échantillon et dudit complexe ;
(d) exposition du complexe à un deuxième anticorps et une partie du deuxième anticorps réagissant avec ledit complexe, l'un desdits premier et deuxième anticorps étant sélectionné parmi le groupe composé d'anticorps polyclonaux spécifiques à l'antigène de H. pylori, d'une pluralité d'anticorps monoclonaux spécifiques à l'antigène de H pylori et de mélanges de ceux-ci, et l'autre étant un anticorps monoclonal spécifique au genre Helicobacter ou Campylobacter, l'un desdits premier et deuxième anticorps étant lié à un excipient solide et l'autre étant marqué par un agent de détection ; et
(e) détection de la quantité d'anticorps marqué et, par la suite, analyse de la présence de l'antigène de H pylori dans ledit échantillon de selles

2. Procédé selon la revendication 1, dans lequel le premier anticorps est lié à un excipient solide et le deuxième anticorps est marqué par un agent de détection

3. Procédé selon la revendication 1, dans lequel le premier anticorps est marqué par un agent de détection et le deuxième anticorps est lié à un excipient solide

4. Procédé selon la revendication 1, dans lequel le diluant d'échantillon est un diluant à base de protéines

5. Procédé selon la revendication 1, dans lequel le premier anticorps est ledit anticorps monoclonal spécifique au genre et ledit deuxième anticorps est sélectionné parmi le groupe composé d'anticorps polyclonaux, d'une pluralité d'anticorps monoclonaux et de mélanges de ceux-ci

6. Procédé selon la revendication 4, dans lequel le diluant d'échantillon contient une protéine sélectionnée parmi le groupe composé du sérum de veau foetal, du sérum de chèvre normal, du sérum de cobaye, du sérum de cheval, de la caséine, de l'albumine, de la gélatine et de l'albumine de sérum bovin

7. Procédé selon la revendication 1, dans lequel, après avoir été exposé au deuxième anticorps, le complexe est lavé à l'aide d'une solution tampon qui réduit la réactivité croisée ou améliore de toute autre manière la spécificité de l'essai

8. Procédé selon la revendication 5, dans lequel le deuxième anticorps est un anticorps polyclonal obtenu en sensibilisant un mammifère produisant des anticorps avec des cellules de H pylori

9. Procédé selon la revendication 3, dans lequel ledit agent de détection est sélectionné parmi le groupe composé de la phosphatase alcaline, la bêta-galactosidase et la peroxydase du raifort.

10. Procédé selon la revendication 7, dans lequel ladite solution de lavage est une solution saline tamponnée au phosphate

11. Procédé d'analyse de présence de H pylori dans un échantillon de selles, comprenant les étapes suivantes :
(a) dispersion d'un échantillon de selles dans un diluant ;
(b) mise de l'échantillon de selles dans le diluant au contact d'un premier anticorps réactif à l'antigène de H. pylori lié à un excipient solide et d'un deuxième anticorps marqué réactif au H pylori afin de former un complexe des anticorps et de l'antigène, l'un desdits premier et deuxième anticorps étant sélectionné parmi le groupe composé d'anticorps polyclonaux spécifiques au H pylori, d'une pluralité d'anticorps monoclonaux spécifiques à l'antigène de H pylori et de mélanges de ceux-ci, et l'autre étant un anticorps monoclonal spécifique au genre Helicobacter ou Campylobacter ;
(c) séparation dudit échantillon et dudit complexe ;
(d) détection de l'anticorps marqué et, par la suite, analyse de la présence de l'antigène de H pylori dans ledit échantillon de selles.

12. Procédé d'analyse de présence de H pylori dans un échantillon de selles, comprenant les étapes suivantes :
(a) dispersion d'un échantillon de selles dans un diluant d'échantillon ;
(b) mise de l'échantillon de selles dans le diluant au contact d'un anticorps monoclonal spécifique au genre Helicobacter ou Campylobacter lié à un excipient solide pour former un complexe de l'anticorps et de l'antigène ;
(c) séparation dudit échantillon et dudit complexe ;
(d) mise du complexe antigène-anticorps formé à l'étape (b) au contact d'un anticorps primaire spécifique à l'antigène de H pylori obtenu auprès d'une espèce produisant des anticorps, afin de produire un complexe anticorps antigène-anticorps ;
(e) élimination de l'anticorps primaire absent du complexe de l'étape (c) ;
(f) mise du complexe anticorps antigène-anticorps formé à l'étape (e) au contact d'un anticorps secondaire, ledit anticorps secondaire étant un anticorps destiné à l'espèce produisant des anticorps, dans lequel ledit deuxième anticorps forme un complexe avec ledit complexe anticorps antigène-anticorps ; et
(g) analyse de la présence de l'antigène de H. pylori dans ledit échantillon de selles

13. Kit permettant d'analyser la présence de H pylori dans un échantillon de selles comprenant une plaque à puits à laquelle est lié un anticorps monoclonal spécifique au genre pour l'antigène de H pylori, un diluant d'échantillon à base de protéines et une pluralité d'anticorps marqués spécifiques à l'antigène de H. pylori
